# EUROPÄISCHE PATENTSCHRIFT

(11) **EP 3 496 605 B1**
(45) Veröffentlichungstag und Bekanntmachung des Hinweises auf die Patenterteilung: **12.06.2024**
(21) Anmeldenummer: 17751378.5
(22) Anmeldetag: 08.08.2017
(51) Int. Cl.: A61B 5/087, A61B 5/09, A61B 5/083, A61B 5/024, A61B 5/097, G01F 1/36

(54) **SPIROERGOMETRIEVORRICHTUNG**
SPIROERGOMETRY APPLIANCE
DISPOSITIF D'ERGOSPIROMÉTRIE

(30) Priorität: 08.08.2016 DE 102016214702
(43) Veröffentlichungstag der Anmeldung: 19.06.2019
(73) Patentinhaber: Sicada Holding GmbH, 86825 Bad Wörishofen (DE)
(72) Erfinder: KNESTEL, Markus, 87496 Hopferbach (DE)
(74) Vertreter: MERH-IP Matias Erny Reichl Hoffmann Patentanwälte PartG mbB
(86) Internationale Anmeldenummer: PCT/EP2017/070097
(87) Internationale Veröffentlichungsnummer: WO 2018/029205

(56) Entgegenhaltungen:
- WO-A1-2015/117046
- US-A1- 2003 065 275
- US-A1- 2009 227 887
- US-A1- 2011 295 140
- US-A1- 2011 302 992
- US-A1- 2012 203 128
- Wikipedia: "Atemschutzmaske", , 12 July 2016 (2016-07-12), XP93091103, Retrieved from the Internet: URL:https://de.wikipedia.org/w/index.php?t itle=Atemschutzmaske&oldid=156082155 [retrieved on 2023-10-12]

## Beschreibung

Die vorliegende Erfindung betrifft eine Spiroergometrievorrichtung zur Erfassung von Parametern eines Atemgases wie beispielsweise die O₂-Konzentration oder die CO₂-Konzentration.

Mobile Ergospirometriegeräte, z. B. für Belastungsuntersuchungen beim Menschen, sind seit einigen Jahren bekannt. Mithilfe derartiger Systeme lassen sich Analysen direkt auf dem Sport- oder Arbeitsplatz unter natürlichen Bedingungen und Belastungssituationen durchführen. Über Telemetrieeinheiten werden Messdaten in Echtzeit an einen Personal Computer oder ein Notebook übertragen, wobei eine entsprechende Steuerung des Trainings- oder Übungsverlaufes nach Auswertung der Daten am Personal Computer möglich ist. Durch derartige Geräte wurden neue Anwendungsgebiete in der Leistungsdiagnostik in der Arbeits-, Sport- und Rehabilitationsmedizin erschlossen.

Ein Ergospirometriesystem ist beispielsweise aus der DE 19960257 C1 bekannt. Insbesondere wird ein Ergospirometriesystem für Tiere, wie Kamele oder Pferde, beschrieben. Über trichterförmige oder zylindrische Atemgasmasken und mit Gasvolumenstrom- oder Mengensensoren, sowie eine Messeinheit mit Sensoren zur Bestimmung der CO₂ oder O₂-Konzentration im Atemgas, werden die Werte nach dem Mischkammer- oder dem Breath-by-Breath-Prinizip ermittelt und im Anschluss über Signalübertragungsmittel zur Messwertweiterverarbeitung, Darstellung und Analyse zu einer Basisstation übermittelt. Zur Bestimmung des Volumenstroms sind Ultraschallwandler vorgesehen.

In der WO 2015/117046 A1 wird ein mobiles Spirometer gezeigt. Dabei besteht das System aus einem Mundstück, einem tubulären Gehäuse und im tubulären Gehäuse liegende Mess- und Prozessmodule. Das Messmodul beinhaltet wiederum verschiedene Sensoren und ist in der Lage gewonnene Sensorendaten als elektrisches Signal an das Prozessmodul weiterzuleiten.

In der US 2003/0065275 A1 wird ein mobiles Kalorimetersystem beschrieben, welches aus einem wegwerfbaren Abschnitt und einem, mit diesem verbundenen, wiederbenutzbaren Abschnitt besteht. Atemgas wird über den wegwerfbaren Abschnitt in eine Leitungsbahn des wiederbenutzbaren Abschnitts abgegeben. Darüber hinaus werden über im wiederbenutzbarem Abschnitt integrierte Sensoren Eigenschaften (Temperatur, Druck und Feuchtigkeit) des Atemgases gemessen.

In der US 2009/0227887 A1 wird ein leichter, tragbarer Stoffwechsel-Analyseumwandler beschrieben, welcher eine Vielzahl analoger Sensoren, mitunter einen galvanischen O₂-Sensor und einen Differenzdrucksensor beinhaltet.

Die US2012/0203128 A1 betrifft ein mobiles Atmungsraten-Messgerät, welches das Ein- und Ausatmen eines Subjektes durch die Analyse des Atemgases, mithilfe von in einem Detektorring implementierten, akustischen Verstärkungssensoren, integrierten, optischen CO₂-, O₂- oder Alkoholsensoren oder mechanischen Windfahnen, erfasst.

Die 2011/0302992 A1 hingegen beschreibt ein stationäres Ergospirometriesystem, welches die O₂-Aufnahme und/oder CO₂-Produktion eines Subjektes auf Grundlage der Resonanz-Absorptionsspektroskopie besti m mt.

Auch die DE 19953866 B4 betrifft ein mobiles Ergospirometriesystem mit einer Messeinheit, welche am Probanden fixierbar ist und Gasvolumen- sowie Mengensensoren zur Bestimmung der CO₂/O₂-Konzentration im Atemgas umfasst. Über einen Signalverarbeitungsprozessor und ein Telemetriemodul sowie eine computergestützte Basisstation mit Telemetrieeinheit zum Aufbau einer drahtlosen Verbindung mit dem Telemetriemodul können von der Basisstation aus online mit dem Probanden Informationen oder Aufforderungen zur Bedienung der Messeinheit und/oder zur Gestaltung des Versuchslaufs übermittelt werden.

Eine Aufgabe der vorliegenden Erfindung ist es, eine verbesserte Spiroergometrievorrichtung bereitzustellen, die insbesondere eine einfachere Handhabung und eine verbesserte Erfassung der Messwerte (wie bspw. Volumenstrom, Atemgastemperatur, absolute Feuchte etc.) ermöglichen soll.

Zur Lösung der vorstehend genannten Aufgabe wird eine Spiroergometrievorrichtung gemäß des unabhängigen Patentanspruchs vorgeschlagen. Die abhängigen Ansprüche betreffen vorteilhafte Beispiele der Erfindung.

Die Vorrichtung (Spiroergometrievorrichtung) zur Erfassung von Parametern eines Atemgases umfasst einen Grundkörper, der eine Atemmaske oder ein Mundstück ist und einen Atemgasführungsabschnitt aufweist. Zudem umfasst die Vorrichtung eine Messeinrichtung mit zumindest einem Sensor zum Erfassen von Parametern des Atemgases . Unter Parameter des Atemgases ist auch beispielsweise die CO₂-Konzentration des Atemgases oder O₂-Konzentration des Atemgases zu verstehen. Zudem ist eine Recheneinheit zum Verarbeiten der erfassten Parameter des Atemgases vorgesehen und ein Energiespeicher zur Energieversorgung zumindest der Messeinrichtung und der Recheneinheit. Die Messeinrichtung ist mit einem Sensor, der am Grundkörper vorgesehen ist, zur Erfassung der Parameter direkt in einem Atemgasstrom ausgestattet . Der Atemgasstrom wird dabei durch den Grundkörper hindurchgeführt . Durch die Anordnung des Sensors am Grundkörper kann somit eine in-situ Erfassung der Parameter des Atemgases ermöglicht werden. Basierend auf dieser Ausgestaltung ist es möglich, einen verbesserten Aufbau der Spiroergometrievorrichtung bereitzustellen, so dass eine digitale modular zerlegbare Atemmaske bereitgestellt werden kann, mit welcher direkt im Atemgasstrom die Konzentrationen von CO₂ oder O₂ und auch der Volumenstrom bestimmbar ist. Somit wird ein einfacher Aufbau der Vorrichtung erreicht und gleichzeitig kann die Hygiene verbessert werden.

Die Messeinrichtung umfasst zumindest einen ersten und einen zweiten Laser, wobei die Parameter des Atemgases über Laserspektroskopie ermittelt werden . Parameter sind dabei insbesondere Atemgasdruck, Atemgastemperatur sowie Atemgasvolumenstrom basierend auf einer Druckdifferenz etc. Die Verwendung eines Laser zur Erfassung der Parameter verbessert einerseits die Hygiene des Aufbaus, da eine rundum geschlossene glatte Oberfläche in der Atemmaske oder im Mundstück vorgesehen werden kann, so dass keine Verkeimungen auftreten. Mit dem Atemgas kommt somit nur eine rundum geschlossene glatte Oberfläche in Berührung. Insbesondere kann der Atemgasführungsabschnitt, welcher im Grundkörper vorgesehen sein kann, eine geschlossene und glatte Oberfläche aufweisen entlang der der Atemgasstrom geführt werden kann. Der Atemgasführungsabschnitt, der insbesondere einen probandenseitigen Atemgaseinlass und einen Atemgasauslass aufweist, kann somit zwischen dem Atemgaseinlass und dem Atemgasauslass eine geschlossene Oberfläche aufweisen, so dass der Atemgasführungsabschnitt rohrförmig mit einem Auslass und einem Einlass für das Atemgas ausgebildet ist. Der Aufbau wird somit weiter verbessert und insbesondere vereinfacht und gleichzeitig wird die Lebensdauer durch Verwendung der Laser als Sensor erhöht.

Die Messeinrichtung kann vorteilhaft zudem einen optischen Sensor umfassen, und insbesondere einen NDIR-Sensor (nichtdispensiver Infrarotsensor). Durch einen optischen Sensor ist es möglich den durch beispielsweise einen Laser ausgeleuchteten Atemgasführungsabschnitt zu analysieren, um die O₂ und CO₂-Konzentration mittels Laserspektroskopie zu ermitteln. Besonders bevorzugt ist ein NDIR-Sensor zur Erfassung der CO2 Konzentration vorgesehen und zudem ein weiterer Sensor (bspw. Sensor mit Laser, paramagnetischer Sensor, el.Chem.O2-Sensor, Optischer Sensor der die Fluoreszenz nutzt oder ein Zr02 Sensor) zur Erfassung der 02 Konzentration.

Die Laser können Halbleiterlaser sein, wobei die Laser insbesondere Diodenlaser, DFB-Laser, IC-Laser und/oder QC-Laser sind. Durch Verwendung der Halbleiterlaser und der genannten Lasertypen ist es möglich eine hohe Lebensdauer und somit eine Verbesserung des Aufbaus zu erreichen. Zudem sind diese Laser leichte robuste Halbleiterbauteile, wodurch auch der Aufbau vereinfacht werden kann. Die genannten Halbleiterlaser ermöglichen auch eine verbesserte Präzision bei der Ermittlung der Parameter.

Die Vorrichtung kann zudem derart ausgestaltet sein, dass der Druck, die Temperatur und der Volumenstrom des Atemgases über die spektrale Auswertung des Linienprofils von Absorptionslinien des Atemgases ermittelt werden kann, wobei die spektrale Auswertung insbesondere durch die Recheneinheit durchgeführt werden kann. Diese Ausgestaltung ermöglicht somit die Erfassung von verschiedensten Parametern und zusätzlich auch des Drucks und der Temperatur des Atemgases über die spektrale Auswertung der Absorptionslinien des Atemgases durch Einsatz der oben genannten Laser. Der Aufbau wird somit insofern vereinfacht, da keine separaten zusätzlichen Sensoren nötig sind. Zudem erlaubt die erfindungsgemäße Messeinrichtung eine hohe Detektionsgenauigkeit.

Der Atemgasführungsabschnitt ist rohrförmig ausgebildet und ein erster Laser und ein zweiter Laser leuchten den Atemgasführungsabschnitt aus und im Bereich des Atemgasführungsabschnitts ist zudem zumindest ein Detektor zur Messung der Absorption vorgesehen. Bevorzugt können die Laser dabei unterschiedliche Wellenlängen aufweisen. Durch eine solche erfindungsgemäße Ausgestaltung kann die Hygiene verbessert werden, da Verkeimungen vermindert werden und die Kondensationsproblematik kann zudem verbessert werden. Zudem wird ein leichter und robuster Aufbau der Vorrichtung erreicht. Die Verwendung von Laserdioden verbessert die Lebensdauer des gesamten Aufbaus. Zudem kann durch die Messung über zwei Laser eine hygienische und keimfreie Messung ermöglicht werden. Die Bestimmung von beispielsweise O₂ und CO₂ kann dabei über Laserspektroskopie erfolgen.

Der erste Laser, der zweite Laser und/oder der Detektor (oder mehrere Detektoren) sind flexibel am Umfang des Atemgasführungsabschnitts vorgesehen, so dass die Position relativ zum Atemgasführungsabschnitt veränderbar ist. Durch die flexible Vorsehung der Laser und/oder Detektoren ist es möglich, einen verbesserten Aufbau bereitzustellen, der flexibel eingesetzt werden kann, da der Messbereich variiert werden kann und zudem kann der Aufbau vereinfacht werden, da zur Erweiterung des Messbereichs keine zusätzlichen weiteren Sensoren nötig sind. Insbesondere kann diese flexible Ausgestaltung dadurch erreicht werden, indem die optische Pfadlänge der Laser variiert wird.

Die Recheneinheit und/oder der Energiespeicher können am Grundkörper vorgesehen sein. Durch diese Ausgestaltung kann ein einfacher und kompakter Aufbau der Ergospirometrievorrichtung erreicht werden. Die Recheneinheit und/oder der Energiespeicher können zudem modular ausgestaltet sein, so dass die Spiroergometrievorrichtung einen modularen Energiespeicher, eine modulare Recheneinheit und/oder eine modulare Messeinrichtung umfasst. Diese modularen Bauteile können auf einfache Weise ausgetauscht werden, da einfach das jeweilige Modul ersetzt werden kann. Über vorgesehene Modulsteckplätze an der Spiroergometrievorrichtung bzw. an deren Grundkörper ist es somit möglich auf einfache Weise die modulare Messeinrichtung, die modulare Recheneinheit und/oder den modularen Energiespeicher auszutauschen. Bevorzugt ist ein Austausch der jeweiligen Komponenten der modular aufgebauten Spiroergometrievorrichtung ohne Einsatz von Schrauben möglich, da die Module lediglich in den Grundkörper aufgeklemmt oder eingesteckt werden. Eine flexible und schnelle Anpassung der Spiroergometrievorrichtung wird dadurch ermöglicht.

Somit kann beispielsweise zur Verbesserung des Energiespeichers dieser einfach ausgetauscht werden und beispielsweise durch einen Energiespeicher mit einer höheren Kapazität ersetzt werden. Da die Recheneinheit und/oder der Energiespeicher direkt am Grundkörper vorgesehen sein können, ist es zudem nicht nötig lange Kabel oder Leitungen zum Grundkörper zu führen, da über den kompakten Aufbau mit direkter Anordnung der Recheneinheit und/oder des Energiespeichers am Grundkörper ein einfacher Aufbau der Spiroergometrievorrichtung erreicht werden kann. Zudem wird auch keine Pumpe zum Absaugen des Atemgases benötigt.

Die Vorrichtung kann zudem eine Übertragungseinrichtung zur Übertragung von Daten der Recheneinheit und/oder des Sensors oder der Sensoren erfassen. Somit wird eine Übertragung der Parameter bzw. der verarbeiteten Parameter zu einer Basisstation ermöglicht. Die Basisstation kann dabei beispielsweise ein Anzeigemittel umfassen (Bildschirm, Display), so dass im Betrieb der Vorrichtung die Parameter und/oder verarbeiteten Parameter angezeigt werden können.

Die Übertragungseinrichtung kann am Grundkörper vorgesehen sein und die Übertragung kann über Funk stattfinden.

Die Messeinrichtung kann als Parameter zumindest einen der folgenden Werte umfassen: die CO₂-Konzentration des Atemgases, die O₂-Konzentration des Atemgases, den Volumenstrom des Atemgases, die Atemgasfeuchte, die Umgebungstemperatur, den Atemgasdruck. Zur Bestimmung der O₂ und CO₂-Konzentration umfasst die Messeinrichtung dabei insbesondere zwei Lasereinrichtungen und zumindest einen Detektor. Die O₂-Konzentration kann mit einem Laser mit einer Wellenlänge von ca. 760nm detektiert. Der Druck, die Temperatur, der Volumenstrom und die Gasfeuchte können über die spektrale Auswertung des Linienprofils der Absorptionslinien des jeweiligen Gases ermittelt werden, und bevorzugt wird dies durch Anordnung von nur zwei Lasern erreicht. Somit genügt die Auswertung einer Absorptionslinie von CO₂ oder O₂. Die Gasfeuchte kann durch geeignete Wahl des CO2 Lasers bestimmt werden.

Bei Verwendung eines Wirkdruck-Stauverfahrens kann der Volumenstrom des Atemgases durch entsprechende Anordnungen der Laser mit den jeweiligen Detektoren erreicht werden. Durch die Verwendung der Laser mit den entsprechenden Detektoren ist es somit möglich die benötigte Anzahl der benötigten Sensoren zu reduzieren und den Aufbau zu verbessern.

Die Auswertung und/oder die Analyse der erfassten Parameter der Messeinrichtung kann über die Recheneinheit durchgeführt werden. Die Vorrichtung ermöglicht somit nicht nur die Ermittlung der einzelnen Messwerte der verschiedenen Konzentrationen im Atemgas oder der Temperatur und des Drucks, sondern zudem auch eine direkte Auswertung und Analyse der Messwerte bzw. eine Verarbeitung der Parameter. Somit kann die erfindungsgemäße Ergospirometrievorrichtung auch beispielsweise einen zeitlichen Verlauf der jeweiligen Parameter aufzeichnen bzw. die Parameter weiterverarbeiten. Insbesondere ist es somit möglich, eine Leistungsdiagnostik direkt durch die Vorrichtung durchzuführen. Bevorzugt ist es auch möglich, eine Evaluation von Therapieprogrammen, Training, Trainingsplan und die Steuerung des Trainings durchzuführen. Zudem kann eine Korrelation mit Normwerten, eine erreichte Leistung in einer bestimmten Zeit, die Menge an Atemvolumen, sowie die Mengen aufgenommenem O₂ und abgegebenem CO₂ erfasst werden.

Die Vorrichtung kann modular zerlegbar aufgebaut sein. Über ein modulares Baukastensystem kann die Vorrichtung somit individuellen Bedürfnissen angepasst werden. So ist zum Beispiel der Austausch der Recheneinheit jederzeit möglich. Somit wird ein verbesserter Aufbau der Ergospirometrievorrichtung erreicht und die Flexibilität und Austauschbarkeit der einzelnen Komponenten erhöht.

Die Vorrichtung kann derart ausgestaltet sein, dass der Grundkörper an einem Probanden über Fixiermittel fixierbar ist und die Recheneinheit, Messeinrichtung und der Energiespeicher direkt am Grundkörper aufgenommen sind, wobei der Grundkörper den Atemgasführungsabschnitt zur Führung des Atemgasstroms aufweisen kann mit einem probandenseitigen Atemgaseinlass und einem Atemgasauslass. Der Sensor kann im Atemgasführungsabschnitt vorgesehen sein. Durch diese Ausgestaltung kann der Aufbau weiter verbessert werden.

Die Vorrichtung kann zudem einen Datenspeicher aufweisen, welcher am Grundkörper angeordnet sein kann und die Recheneinheit kann Spiroergometriegrößen aus den ermittelten Messwerten/Parametern berechnen und diese im Datenspeicher speichern. Durch den Datenspeicher ist es somit möglich, die Messwerte direkt zu speichern, so dass die Vorrichtung auch bei Einsatz im Freien ohne Verwendung einer Basisstation eingesetzt werden kann. Zudem wird der gesamte Aufbau der Ergospirometrievorrichtung vereinfacht, da kein externer Speicher benötigt wird, sondern der im Grundkörper vorgesehene Datenspeicher die Daten der Vorrichtung speichern kann.

Am Grundkörper kann ein EKG-Modul vorgesehen sein und die Messeinrichtung kann zusätzlich Sensoren zum Erfassen der Herzfrequenz umfassen. Durch diese Ausgestaltung der Ergospirometrievorrichtung ist es möglich, weitere Messwerte zu erfassen und somit die Analyse weiter zu verbessern. Zusätzliche Gerätschaften für das EKG-Modul sind somit nicht benötigt und die Leistungserfassung kann durch einfache Weise unter Verwendung der erfindungsgemäßen Vorrichtung durchgeführt werden. Der Aufbau ist somit einfacher und optimiert im Vergleich zu der Analyse unter Verwendung von verschiedenen, voneinander getrennten Vorrichtungen, nämlich zur Erfassung der O₂ und CO₂-Atemgaskonzentration und einer weiteren Vorrichtung zur Erfassung der Herzfrequenz.

Die Vorrichtung kann zudem derart ausgestalten sein, dass die vom Betrieb der Vorrichtung benötigte Energie ausschließlich über den Energiespeicher bezogen werden kann. Somit wird die Vorrichtung derart ausgestaltet, dass es möglich ist, einen kabellosen Betrieb durchzuführen, wenn der Energiespeicher am Grundkörper direkt vorgesehen ist. Zudem ist es möglich durch Bereitstellung der Energie für den Betrieb der Vorrichtung über den Energiespeicher auf Energiequellen, welche außerhalb der Spiroergometrievorrichtung vorliegen, zu verzichten, so dass der Aufbau für den Betrieb der Spiroergometrievorrichtung vereinfacht werden kann und eine flexiblere Messung auch ortsunabhängig durchgeführt werden kann.

Vorteilhaft kann die Vorrichtung auch eine Messeinrichtung umfassen, welche einen nichtdispersiven Infrarotsensor und/oder einen Zirkoniumdioxidsensor umfasst.

Die Vorrichtung kann zudem einen Generator zum Durchführen von Energy Harvesting umfassen, so dass eine Energiegewinnung aus Stoßimpulsen und/oder Atemgaswärme und/oder Umgebungsbeleuchtung ermöglicht wird und der Energiespeicher dadurch wieder aufgeladen werden kann. Die Vorrichtung kann somit sehr flexibel eingesetzt werden. Der Gesamtaufbau kann dadurch auch vereinfacht werden, da externe Ladegeräte und Ladestationen nicht benötigt werden. Ein Laden der Vorrichtung kann kabellos erfolgen.

Die Messeinrichtung kann einen ersten Sensor zur Erfassung der O₂-Konzentration und der CO₂-Konzentration im Atemgas umfassen und einen zweiten Sensor zur Bestimmung des Volumenstroms des Atemgases. Dies ermöglicht einen besonders einfachen Aufbau der Vorrichtung.

Der zweite Sensor kann eine durch einen Atemgasstrom angetriebene Turbine umfassen. Der zweite Sensor kann zudem ein Sensor sein, der die Durchflussmessung basierend auf einem akustischen, gyroskopischen, magnetisch-induktiven, optischen, thermischen, oder Wirkdruck-Stauverfahren durchführt. Durch den zweiten Sensor kann somit auf einfache Weise der Volumenstrom des Atemgases durch Durchführung der Durchflussmessung durchgeführt werden.

Zur Bestimmung des Volumenstroms des Atemgases über einen Differenzdruck im Atemgasführungsabschnitt kann eine Blende vorgesehen sein, welche zwischen einem ersten Laser und einem zweiten Laser vorgesehen ist. Durch diese einfache Ausgestaltung ist es möglich einen Sensor bereitzustellen, welcher zwei Laser umfasst, und somit die CO₂ und O₂-Konzentration ermitteln kann und zudem über die im Sensor vorgesehene Blende den Differenzdruck und somit den Volumenstrom des Atemgases erfassen kann. Zudem ist es möglich, durch diese Anordnung die absolute Feuchte direkt zu ermitteln. Somit wird dadurch eine besonders einfache Ausgestaltung der Vorrichtung vorgeschlagen.

Die Vorrichtung kann eine mobile Spiroergometrievorrichtung sein. Zudem kann die Vorrichtung auch als Teil eines Ergospirometriesystems vorliegen, welche auch eine Basisstation umfassen kann.

Vorteilhafte Weiterbildungen der beschriebenen Aspekte werden im Folgenden kurz zusammengefasst:
Vorrichtung nach einem der vorhergehenden Aspekte, wobei die Laser Halbleiterlaser sind und die Laser insbesondere, Diodenlaser, DFB-Laser, IC-Laser, und/oder QC-Laser sind.

Vorrichtung nach zumindest einem der vorangehenden Aspekte, wobei die Recheneinheit und/oder der Energiespeicher am Grundkörper vorgesehen ist.

Vorrichtung nach zumindest einem der vorangehenden Aspekte, wobei die Vorrichtung eine Übertragungseinrichtung zur Übertragung von Daten der Recheneinheit und/oder des Sensors umfasst.

Vorrichtung nach dem vorangehenden Aspekte, wobei die Übertragungseinrichtung am Grundkörper vorgesehen ist und die Übertragung über Funk stattfindet.

Vorrichtung nach zumindest einem der vorangehenden Aspekte, wobei die Vorrichtung modular zerlegbar aufgebaut ist. Dies ermöglicht eine besonders einfache Handhabung vor allem bei der Wartung und beim Austausch von Bauteilen.

Vorrichtung nach zumindest einem der vorangehenden Aspekte, wobei am Grundkörper zudem ein EKG-Modul vorgesehen ist und die Messeinrichtung zusätzlich Sensoren zum Erfassen der Herzfrequenz umfasst.

Vorrichtung nach zumindest einem der vorangehenden Aspekte, wobei die Messeinrichtung einen nichtdispersiven Infrarotsensor und/oder einen Zirkoniumdioxidsensor umfasst.

Vorrichtung nach zumindest einem der vorangehenden Aspekte, wobei ein Datenspeicher vorgesehen ist, welcher am Grundkörper angeordnet ist und die Recheneinheit Spiroergometriegrößen aus den ermittelten Parametern berechnet und diese im Datenspeicher speichert.

Spiroergometriesystem mit einer Vorrichtung gemäß zumindest einem der vorangehenden Aspekte.

Vorrichtung nach zumindest einem der vorangehenden Aspekte, wobei die Messeinrichtung einen ersten Sensor zur Erfassung der 02-Konzentration und der CO2-Konzentration im Atemgas umfasst und einen zweiten Sensor zur Bestimmung des Volumenstroms des Atemgases.

Vorrichtung nach einem der vorangegangenen Aspekte, wobei der zweite Sensor eine durch einen Atemgasstrom angetriebene Turbine umfasst oder wobei der zweite Sensor ein Sensor ist der die Durchflussmessung basierend auf einem akustischen, gyroskopischen, magnetisch-induktiven, optischen, thermischen oder Wirkdruck/Stau-Verfahren durchführt.

Vorteilhafte Ausgestaltungen und weitere Details werden im Folgenden anhand verschiedener Ausführungsbeispiele mit Bezug auf schematische Figuren beschrieben. In den schematischen Zeichnungen wird die Erfindung näher erläutert.

### Figuren

- Figur 1:: zeigt einen schematischen Aufbau einer Atemgasmaske mit einer Messeinrichtung zum Erfassen von Parametern des Atemgases;
- Figur 2:: zeigt eine schematische Darstellung des Atemgasführungsabschnitts und der daran angeordneten Laser und Detektoren zur Bestimmung der CO₂-, O₂-, H₂O-Konzentrationen sowie der Temperatur, des Drucks und des Volumendstroms;
- Figur 3:: zeigt eine Atemmaske mit dem Atemgaseinlass und -auslass;
- Figur 4a:: zeigt eine Ausführungsform der vorliegenden Erfindung mit zwei Detektoren und zwei Lasern;
- Figur 4b:: zeigt eine Ausführungsform der vorliegenden Erfindung mit einem Detektor und zwei Lasern;
- Figur 5:: zeigt eine Spiroergometrievorrichtung gemäß der vorliegenden Erfindung mit einer Energy Harvesting Vorrichtung; und
- Figur 6:: zeigt ein Diagramm für die Multiparameterbestimmung.

Im Folgenden werden verschiedene Beispiele detailliert und mit Bezugnahme auf die Figuren beschrieben. Gleiche bzw. ähnliche Elemente werden mit gleichen Bezugszeichen bezeichnet. Die vorliegende Erfindung ist in den Ansprüchen definiert.

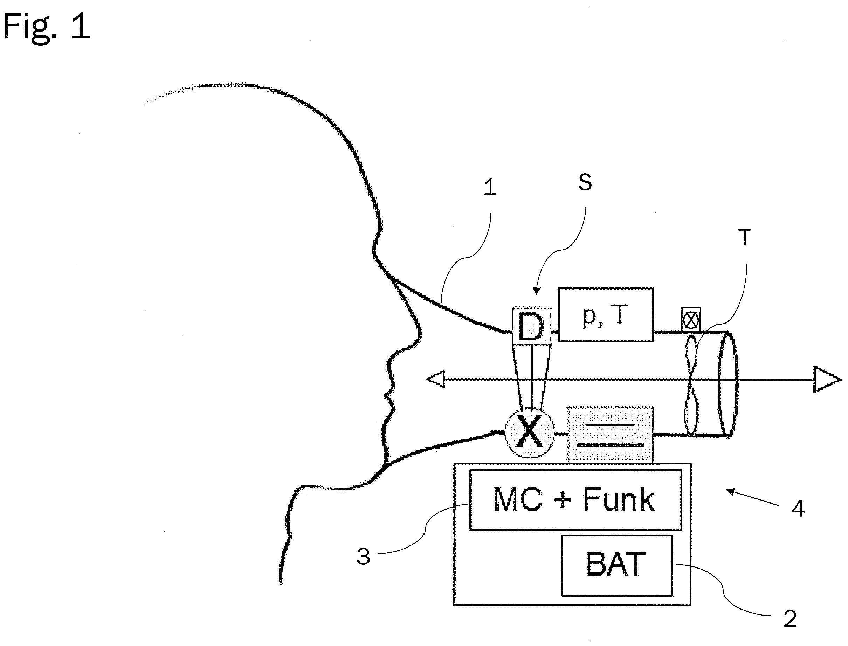

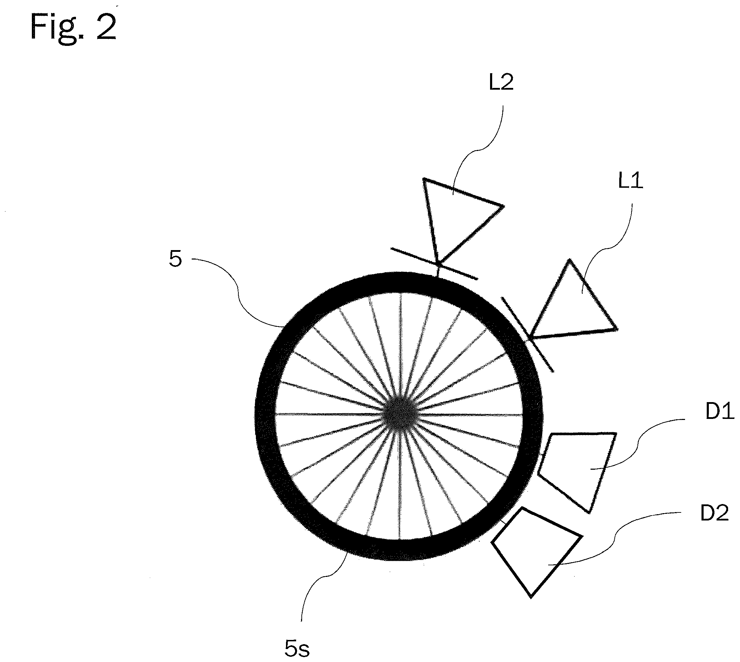

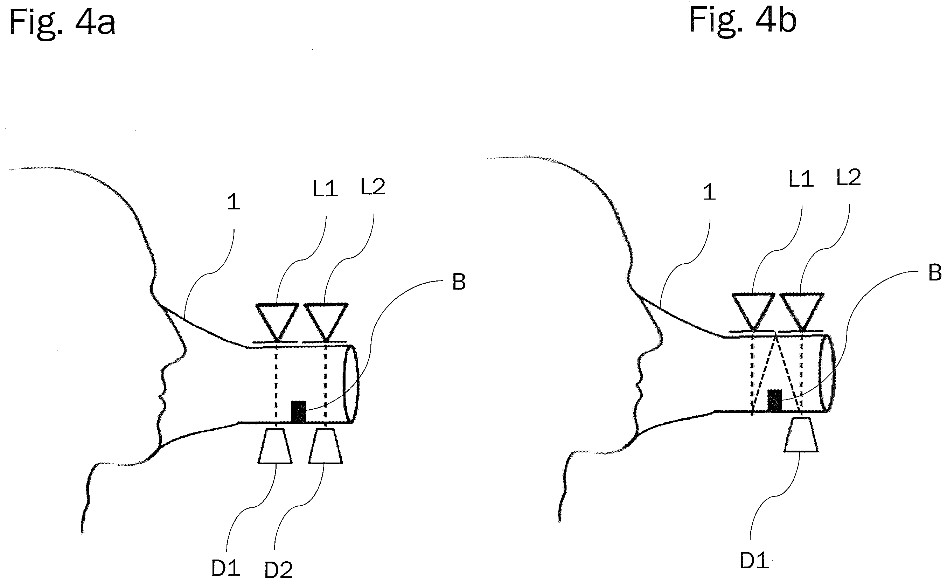

In Figur 1 ist ein schematischer Aufbau einer Spiroergometrievorrichtung dargestellt. An einem Grundkörper 1, welcher in der Ausführungsform in Figur 1 als Atemmaske ausgeführt ist, befinden sich verschiedene Sensoren S. Für die Erfassung des Drucks und der Temperatur können ein Drucksensor und ein Temperatursensor vorgesehen sein. Die Sensoren S sind der Messeinrichtung 4 zugeordnet, welche mit der Recheneinheit 3 in Verbindung steht. Die Recheneinheit 3 wertet die erfassten Messwerte der Messeinrichtung 4 zu den jeweiligen Parametren aus. Zum Betrieb der Recheneinheit 3 ist eine Batterie (Energiespeicher 2) vorgesehen. Anstatt der verschiedenen Sensoren ist es möglich, lediglich über zwei Laser mit zumindest einem Detektor die Parameter (p, V, T, O₂, CO₂ etc.) zu ermitteln, gemäß dem Beispiel in Figuren 2, 4a und 4b. Die komplizierte Auswertung der verschiedensten Sensorarten kann dadurch stark vereinfacht werden, da nunmehr die von den Lasern erzeugten Signale ausgewertet werden müssen. Damit dem Probanden ein Atmen ermöglicht ist, verfügt der Grundkörper über eine Atemgasauslassöffnung, welche gegenüberliegend zum Atemgaseinlass, über welchen das Atemgas des Probanden in den Grundkörper eingeleitet wird, vorgesehen ist. Da die Verarbeitung der erfassten Messwerte direkt in der Spiroergometrievorrichtung durchgeführt werden kann, ist eine Verbindung über Kabel oder Schläuche zu einer Basisstation nicht nötig. Ein Abpumpen des Atemgases über eine Pumpe ist ebenfalls nicht nötig.

Die Atemgasmaske ist derart abgedichtet am Kopf des Probanden angebracht, dass der Atemgasstrom ausschließlich über den Atemgasführungsabschnitt geleitet wird, wobei die Maske Dichtmittel oder Dichtflächen aufweist, welche eine entsprechende Anbringung an den Probanden ermöglichen.

In einer besonders bevorzugten Ausführungsform verfügt die Messeinrichtung 4 über zwei Laser mit insgesamt einem (oder zwei) Detektoren und ein Turbinenrad. Über das Turbinenrad wird der Volumenstrom der Atemluft gemessen und über die zwei Laser und einen Detektor (und Multiplexer) werden die weiteren Parameter (p, T, CO₂, O₂, absolute Luftfeuchte) ermittelt.

Eine weitere Vereinfachung wird durch den Aufbau wie er in Figur 2 dargestellt ist, erreicht. Bei diesem Aufbau wird für die Messeinrichtung ein Sensor bereitgestellt, welcher einen ersten Laser L1 und einen zweiten Laser L2 umfasst, welche am Umfang des Atemgasführungsabschnitts 5 angeordnet sind. Diese Laser leuchten den inneren Bereich des Atemgasführungsabschnitts aus, welcher zumindest teilweise eine reflektierende Oberfläche aufweist und beispielsweise einen Zirkularreflektor umfasst. Der Atemgasführungsabschnitt ist dabei rohrförmig ausgestaltet. Über den ersten Detektor D1 und den zweiten Detektor D2 ist es möglich, die O₂-Konzentration und CO₂-Konzentration des Atemgases zu ermitteln. Insbesondere ist der erste Laser L1 derart ausgeführt, dass er eine Laserstrahlung mit einer Wellenlänge emittieren kann, welche unterschiedlich ist zur Wellenlänge des zweiten Laser L2 emittiert. Mittels Laserspektroskopie ist es somit möglich die O₂ und CO₂-Konzentration basierend auf den unterschiedlichen Wellenlängen zu ermitteln. Der erste Laser L1 emittiert die Strahlung dabei bei einer Wellenlänge von 760nm und der zweite Laser L2 emittiert die Strahlung dabei bei einer Wellenlänge von bevorzugt 2µm oder 4,2µm.

Durch die vorteilhafte Ausgestaltung des Sensors der Messeinrichtung mit dem ersten Laser und dem zweiten Laser ist eine verbesserte Hygiene möglich, da Verkeimungen vermieden werden und, da der Atemgasführungsabschnitt eine rundum geschlossene und glatte Oberfläche bildet und die Messung somit berührungslos möglich ist.

Die Position des ersten Lasers L1 und/oder des zweiten Lasers L2 sind dabei einstellbar am Umfang des Atemgasführungsabschnitts 5. Durch Veränderung der Position der Laser oder auch der Detektoren, ist es möglich den Messbereich des Sensors S zu verändern und zwar durch Variation der optischen Pfadlänge. Alternativ oder zusätzlich kann auch die Detektorposition des ersten Detektors D1 oder des zweiten Detektors D2 verändert werden, um den Messbereich zu beeinflussen (Zirkulationsreflektor). Über eine Vorrichtung, welche die erfindungsgemäße Messeinrichtung umfasst, ist es möglich den Verschleiß zu reduzieren, da keine beweglichen Teile oder Pumpen nötig sind. Die Messung erfolgt somit in-situ und daher direkt in der Atemgasmaske.

Für den Laser zur Bestimmung der CO₂-Konzentration wird bevorzugt ein Diodenlaser verwendet oder eine Leuchtdiode im NIR und MIR-Bereich. Durch die Verwendung von Lasersensorik für die Bestimmung der Atemgasparameter ist zudem eine hohe Lebensdauer der Messeinrichtung erreichbar, da die Laserdioden eine hohe Lebensdauer aufweisen. Zudem ist die Kondensationsproblematik der Spiroergometrievorrichtung reduziert, so dass beispielsweise keine Trocknung des Messgases notwendig ist. Der Aufbau und die Messung werden daher vereinfacht.

Durch Variation der Detektorposition kann die optische Pfadlänge einstellbar sein, wodurch der Messbereich des Sensors eingestellt werden kann. In Figur 2, in der der Atemgasführungsabschnitt im Querschnitt dargestellt ist, ist der innere Bereich des Atemgasführungsabschnitts 5 durch den Laserstrahl des Lasers L1 und des Lasers L2 ausgeleuchtet, wie dies schematisch durch die speichenartigen Linien im Inneren des Atemgasführungsabschnitts 5 dargestellt ist. Zur Erzeugung der Reflektionen ist bevorzugt eine reflektierende Schicht im Abschnitt vorgesehen, wie ein Spiegel o.ä., wobei dies nicht zwingend ist. Die Position des ersten Detektors D1 und/oder zweiten Detektors D2 kann relativ zum Atemgasführungsabschnitt 5 variiert werden, so dass die optische Pfadlänge verlängert oder verkürzt werden kann. Dadurch kann der Messbereich eingestellt werden.

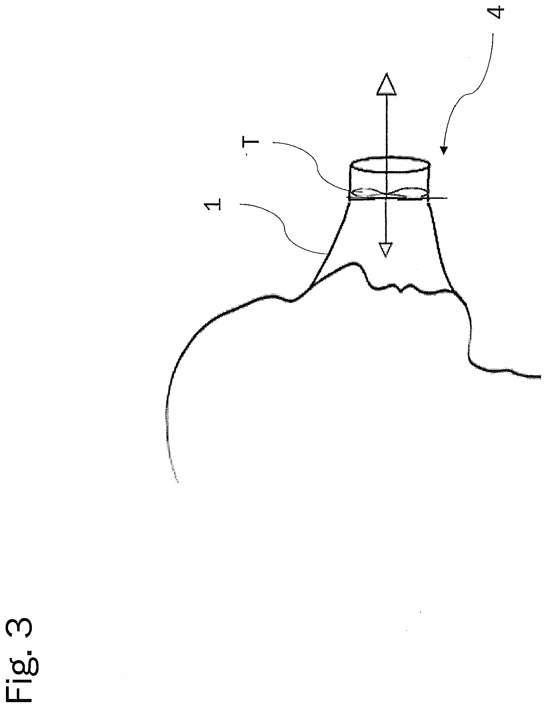

In Figur 3 ist ein Grundkörper dargestellt, der als Atemmaske ausgestaltet ist, wobei die Messeinrichtung 4 zusätzlich einen Sensor umfasst, welcher eine angetriebene Turbine beinhaltet. Über diese vom Atemgas angetriebene Turbine ist es möglich, eine Durchflussmessung durchzuführen und den Volumenstrom des Atemgases zu bestimmen. Die durch die Einatmung und Ausatmung des Probanden erzeugte Strömung des Atemgases ist dabei durch den Pfeil, welcher durch den Grundkörper 1 verläuft, dargestellt.

In Figuren 4a und 4b ist eine besonders vorteilhafte Ausgestaltung der vorliegenden Erfindung dargestellt. Die Spiroergometrievorrichtung gemäß Figur 4a weist einen ersten Laser L1 und einen zweiten Laser L2 auf, welche jeweils einem Detektor D1 und einem zweiten Detektor D2 (nicht zwingend) zugeordnet sind. Der Grundkörper 1 weist zudem einen Atemgasführungsabschnitt auf, der durch den Grundkörper geführt ist und durch welchen das Atemgas zum Probanden und vom Probanden weg geleitet werden kann. Bei Einatmung wird über den vom Probanden abgewandten Teil des Grundkörpers über die erste Öffnung Atemgas zum Probanden zugeführt und bei der Ausatmung des Probanden über diese Öffnung abgeführt. Bevorzugt kann jedoch auch nur ein Detektor vorgesehen sein, gemäß Figur 4b.

Zur Messung der O₂-Konzentration und CO₂-Konzentration ist der erste Laser mit einer ersten Wellenlänge und der zweite Laser mit einer zweiten Wellenlänge ausgestattet, wobei die erste Wellenlänge unterschiedlich ist von der zweiten Wellenlänge. Die vom ersten Laser L1 emittierte Strahlung wird in den Atemgasführungsabschnitt des Grundkörpers 1 geführt und erreicht den ersten Detektor D1. Analog wird die vom zweiten Laser emittierte Strahlung durch den Atemgasführungsabschnitt des Grundkörpers 1 zum Detektor D2 geführt. Alternativ oder zusätzlich ist auch die Strahlenführung auf nur einen einzigen Detektor möglich, gemäß Figur 4b.

Der Grundkörper 1 weist dabei einen rohrförmigen Atemgasabschnitt auf, wobei der erste Laser L1 und der zweite Laser L2 in axialer Richtung entlang der Längsrichtung des Atemgasführungsabschnitts voneinander beabstandet angeordnet sind. Zwischen dem ersten Laser L1 und dem zweiten Laser L2, in axialer Richtung, ist eine Blende B vorgesehen. Die Blende B ist zudem zwischen dem ersten Detektor D1 und dem zweiten Detektor D2 angeordnet. Die jeweiligen Detektoren sind dabei dem ersten und dem zweiten Laser zugeordnet. Die Blende B liegt im Atemgasführungsabschnitt 5 vor und reicht somit in den Atemgasstrom, der durch den Atemgasführungsabschnitt 5 geführt wird. Über die Blende B wird der Atemgasstrom abgelenkt. Der Atemgasstrom, welcher durch den Erfassungsbereich des ersten Sensors (welcher durch den ersten Laser L1 und den ersten Detektor D1 gebildet ist) strömt, wird durch die Blende B abgelenkt (gestaut), so dass im Erfassungsbereich des zweiten Sensors (welcher durch den zweiten Laser L2 und den zweiten Detektor D2 gebildet wird) der Atemgasstrom derart abgelenkt wird, dass ein Differenzdruck erzeugt wird. Dieser Differenzdruck kann durch Anwendung des Wirkdruck-Stauverfahrens für eine Durchflussmessung verwendet werden, um den Volumenstrom des Atemgases, welches sich durch den Atemgasführungsabschnitt bewegt, zu ermitteln. Die Oberfläche des Atemgasführungsabschnitts 5, durch welche das Atemgas geleitet wird, kann zudem durch eine schmutz- und wasserabweisende Oberflächenbeschichtung, wie beispielsweise Polytetrafluorethylen oder ähnlichen Materialien beschichtet sein. Dadurch kann die Hygiene weiter verbessert werden.

Zur Erzeugung der Reflektion ist die Innenseite des rohrförmigen Atemgasabschnitts mit einer reflektierenden Schicht 5s beschichtet oder es können Spiegel vorgesehen sein. (Insbesondere kann eine Aluminiumoxidschicht vorgesehen sein.)

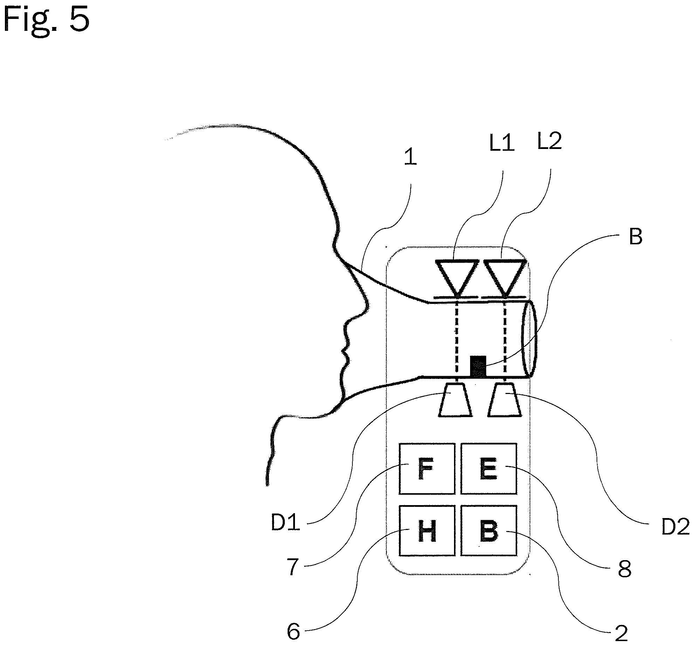

In Figur 5 ist die Spiroergometrievorrichtung wie in Figur 4 dargestellt, wobei zusätzlich ein Funkmodul 7, ein Energy-Harvestingmodul 6, ein Batteriespeicher 2 und die Auswertungselektronik 8 als Teil der Vorrichtung dargestellt sind.

Das Energy-Harvesting-Modul 6 kann insbesondere piezoelektrische Kristalle umfassen, welche bei Krafteinwirkung elektrische Spannungen erzeugen. Alternativ oder zusätzlich können thermoelektrische Generatoren und pyroelektrische Kristalle vorgesehen sein, welche aus den Temperaturunterschieden zwischen dem Atemgas und der Umgebungstemperatur elektrische Energie gewinnen. Das Energy Harvesting- Modul kann zudem Photovoltaik-Elemente umfassen, um Energie aus der Umgebungsbeleuchtung zu gewinnen. Damit wird ein vereinfachter Aufbau des Spiroergometriesystems bereitgestellt, mit dem ein Aufladen des Energiespeichers 2 direkt über das Energy-Harvesting-Modul 6 durchgeführt werden kann ohne aufwendige externe Aufladegeräte zu benötigen. Ein Aufladen der Vorrichtung kann insbesondere über induktives Laden erfolgen und somit drahtlos.

Die verschiedenen Module sind im Ausführungsbeispiel der Figur 5 zudem direkt am Grundkörper 1 vorgesehen, so dass eine kompakte und einfache Spiroergometrievorrichtung bereitgestellt werden kann.

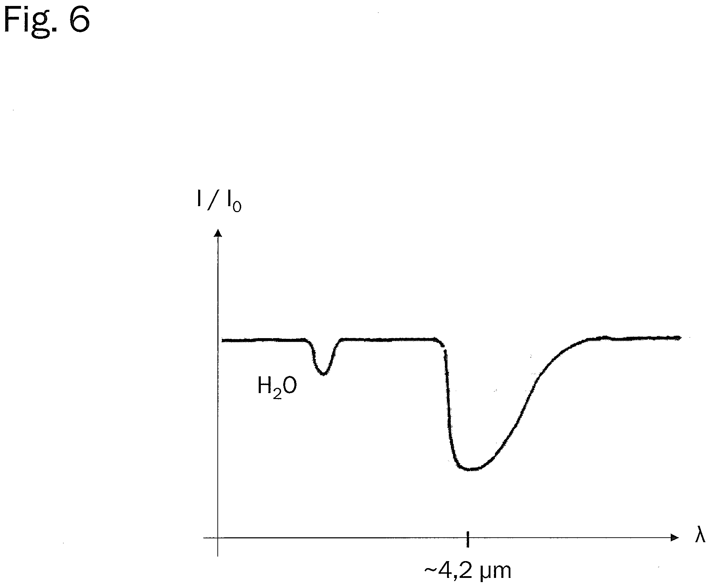

Die Ermittlung der verschiedenen Parameter (O₂, CO₂, T, p, Δp) erfolgt über die Auswertung der Spektraldaten der Strahlung der Laser (Spektrallinien, Absorptionslinien). Dazu wird eine Multiparameterbestimmung durchgeführt. Wie in Figur 6 dargestellt, verändert sich die gemessene Intensität I (welche in der Figur normiert wurde) über die Wellenlänge λ. Durch Anwendung von Laserspektroskopieverfahren unter Verwendung von bekannten Spektralmodellen der Gase können die gesuchten Parameter für die Multiparameterbestimmung ermittelt werden. Beispielsweise kann durch Bestimmung der Verschmälerung der Kurve ein Druck (Druckdifferenz) des Atemgases berechnet werden. Bei Verwendung der Blende B kann über die Druckdifferenz auch der Volumenstrom bestimmt werden.

Vorliegende Merkmale, Komponenten und spezifische Details können ausgetauscht und/oder kombiniert werden, in Abhängigkeit des geforderten Verwendungszwecks. Etwaige Modifikationen die im Bereich des Wissens des Fachmanns liegen, werden mit der vorliegenden Beschreibung implizit offenbart.

## Patentansprüche

1. Vorrichtung zur Erfassung von Parametern eines Atemgases, mit
- einem Grundkörper (1), der eine Atemmaske oder ein Mundstück ist und einen Atemgasführungsabschnitt (5) aufweist,
- einer Messeinrichtung (4) zum Erfassen von Parametern des Atemgases,
- einer Recheneinheit (3) zum Verarbeiten der erfassten Parameter des Atemgases, und
- einem Energiespeicher (2) zur Energieversorgung zumindest der Messeinrichtung (4) und der Recheneinheit (3);
wobei
die Vorrichtung eine mobile Spiroergometrievorrichtung ist,
die Messeinrichtung (4) zumindest einen ersten Laser (L1) und einen zweiten Laser (L2) zur Ausleuchtung des Atemgasführungsabschnitts (5) und einen Detektor (D) zur Messung der Absorption umfasst, und
die Messeinrichtung (4) am Grundkörper (1) vorgesehen ist zur Erfassung der Parameter direkt in einem Atemgasstrom, der durch den Grundkörper (1) geführt wird, sodass eine in-situ Erfassung der Parameter des Atemgases ermöglicht wird; wobei
der Atemgasführungsabschnitt (5) rohrförmig ausgebildet ist und
der zumindest erste Laser (L1), der zweite Laser (L2) und/oder der Detektor (D) flexibel am Umfang des Atemgasführungsabschnitts (5) vorgesehen sind, so dass die Position des zumindest ersten Lasers (L1), des zweiten Lasers (L2) und/oder des Detektors (D) relativ zum Atemgasführungsabschnitts (5) veränderbar ist; und
der zumindest erste Laser (L1), der zweite Laser (L2) und/oder der Detektor (D) eingerichtet sind, den Messbereich der Messeinrichtung (4) durch Variierung der Pfadlänge des ersten Lasers (L1) und/oder des zweiten Lasers (L2) zu variieren;
wobei der innere Bereich des Atemgasführungsabschnitts (5) zumindest teilweise eine reflektierende Oberfläche aufweist und einen Zirkularreflektor umfasst und der erste Laser (L1) und der zweite Laser (L2) eingerichtet sind den inneren Bereich des Atemgasführungsabschnitts (5) auszuleuchten.

2. Vorrichtung nach Anspruch 1, wobei die Auswertung und/oder Analyse der erfassten Parameter der Messeinrichtung (4) über die Recheneinheit (3) durchführbar ist.

3. Vorrichtung nach zumindest einem der Ansprüche 1 oder 2, wobei die Messeinrichtung (4) einen Sensor umfasst, der zumindest die zwei Laser aufweist, wobei die Parameter des Atemgases über Laserspektroskopie ermittelt werden.

4. Vorrichtung nach Anspruch 3, wobei der Sensor zwei Laser umfasst welche unterschiedliche Wellenlängen aufweisen.

5. Vorrichtung nach Anspruch 3 , wobei der Druck und die Temperatur des Atemgases über die spektrale Auswertung des Linienprofils von Absorptionslinien des Atemgases ermittelt werden und wobei die spektrale Auswertung insbesondere durch die Recheneinheit (3) durchgeführt wird.

6. Vorrichtung nach zumindest einem der vorangehenden Ansprüche, wobei die Messeinrichtung (4) als Parameter zumindest einen der folgenden Parameter erfasst: die CO₂-Konzentration des Atemgases, die O₂-Konzentration des Atemgases, den Volumenstrom des Atemgases, die Atemgasfeuchte, die Umgebungstemperatur, der Atemgasdruck.

7. Vorrichtung nach zumindest einem der vorangehenden Ansprüche, wobei der Grundkörper (1) an einem Probanden über Fixiermittel fixierbar ist und die Recheneinheit (3), Messeinrichtung (4) und der Energiespeicher (2) am Grundkörper (1) aufgenommen sind, wobei der Grundkörper (1) den Atemgasführungsabschnitt zur Führung des Atemgasstroms aufweist mit einem probandenseitigen Atemgaseinlass und einem Atemgasauslass und wobei der Sensor (S) im Atemgasführungsabschnitt vorgesehen ist.

8. Vorrichtung nach zumindest einem der vorangehenden Ansprüche, wobei die Vorrichtung die für den Betrieb benötigte Energie ausschließlich über den Energiespeicher (2) bezieht.

9. Vorrichtung nach zumindest einem der vorangehenden Ansprüche, wobei die Messeinrichtung (4) einen nichtdispersiven Infrarotsensor und/oder einen Zirkoniumdioxidsensor umfasst.

10. Vorrichtung nach zumindest einem der vorangehenden Ansprüche, wobei ein Generator zum Durchführen von Energy Harvesting vorgesehen ist, sodass eine Energiegewinnung aus Stoßimpulsen und/oder Atemgaswärme und/oder Umgebungsbeleuchtung ermöglicht wird.

11. Vorrichtung nach zumindest einem der vorangehenden Ansprüche, wobei zur Bestimmung des Volumenstroms des Atemgases über einen Differenzdruck im Atemgasführungsabschnitt eine Blende vorgesehen ist welche zwischen dem ersten Laser (L1) und dem zweiten Laser (L2) vorgesehen ist.

## Claims

1. Device for detecting parameters of a breathing gas, comprising
- base body (1) which is a breathing mask or a mouthpiece and has a breathing gas guide section (5),
- measuring device (4) for detecting parameters of the breathing gas,
- computing unit (3) for processing the detected parameters of the breathing gas, and
- an energy store (2) for supplying energy to at least the measuring device (4) and the computing unit (3);
wherein
the device is a mobile spiroergometry device,
the measuring device (4) comprises at least a first laser (L1) and
a second laser (L2) for illuminating the breathing gas guide section (5) and a detector (D) for measuring the absorption, and
the measuring device (4) is provided on the base body (1) for detecting the parameters directly in a breathing gas flow which is guided through the base body (1), so that an in-situ detection of the parameters of the breathing gas is made possible; wherein
the breathing gas guide section (5) is tubular and
the at least first laser (L1), the second laser (L2) and/or the detector (D) are provided flexibly on the circumference of the breathing gas guide section (5), so that the position of the at least first laser (L1), the second laser (L2) and/or the detector (D) relative to the breathing gas guide section (5) is variable; and
the at least first laser (L1), the second laser (L2) and/or the detector (D) are configured to vary the measuring range of the measuring device (4) by varying the path length of the first laser (L1) and/or the second laser (L2);
wherein the inner region of the breathing gas guide section (5) has at least partially a reflective surface and comprises a circular reflector and the first laser (L1) and the second laser (L2) are configured to illuminate the inner region of the breathing gas guide section (5).

2. Device according to claim 1, wherein the evaluation and/or analysis of the detected parameters of the measuring device (4) can be carried out via the computing unit (3).

3. Device according to at least one of claims 1 or 2, wherein the measuring device (4) comprises a sensor which has at least the two lasers, wherein the parameters of the breathing gas are determined via laser spectroscopy.

4. Device according to claim 3, wherein the sensor comprises two lasers which have different wavelengths.

5. Device according to claim 3, wherein the pressure and the temperature of the breathing gas are determined via the spectral evaluation of the line profile of absorption lines of the breathing gas and wherein the spectral evaluation is carried out in particular by the computing unit (3).

6. Device according to at least one of the preceding claims, wherein the measuring device (4) detects as parameters at least one of the following parameters: the CO₂ concentration of the breathing gas, the O₂ concentration of the breathing gas, the volume flow of the breathing gas, the breathing gas humidity, the ambient temperature, the breathing gas pressure.

7. Device according to at least one of the preceding claims, wherein the base body (1) can be fixed to a subject via fixing means and the computing unit (3), measuring device (4) and the energy store (2) are accommodated on the base body (1), wherein the base body (1) has the breathing gas guide section for guiding the breathing gas flow with a subject-side breathing gas inlet and a breathing gas outlet and wherein the sensor (S) is provided in the breathing gas guide section.

8. Device according to at least one of the preceding claims, wherein the device obtains the energy required for operation exclusively via the energy store (2).

9. Device according to at least one of the preceding claims, wherein the measuring device (4) comprises a non-dispersive infrared sensor and/or a zirconium dioxide sensor.

10. Device according to at least one of the preceding claims, wherein a generator is provided for carrying out energy harvesting, so that an energy recovery from shock pulses and/or breathing gas heat and/or ambient lighting is made possible.

11. Device according to at least one of the preceding claims, wherein a diaphragm is provided for determining the volume flow of the breathing gas via a differential pressure in the breathing gas guide section, which diaphragm is provided between the first laser (L1) and the second laser (L2).

## Revendications

1. Dispositif de détection de paramètres d'un gaz respiratoire, comprenant
- un corps de base (1) qui est un masque respiratoire ou un embout buccal et qui présente une section de guidage de gaz respiratoire (5),
- un dispositif de mesure (4) pour détecter des paramètres du gaz respiratoire,
- une unité de calcul (3) pour traiter les paramètres détectés du gaz respiratoire, et
- un accumulateur d'énergie (2) pour alimenter en énergie au moins le dispositif de mesure (4) et l'unité de calcul (3) ;
dans lequel
le dispositif est un dispositif de spirométrie mobile,
le dispositif de mesure (4) comprend au moins un premier laser (L1) et un deuxième laser (L2) pour éclairer la section de guidage de gaz respiratoire (5) et un détecteur (D) pour mesurer l'absorption, et
le dispositif de mesure (4) est prévu sur le corps de base (1) pour détecter les paramètres directement dans un flux de gaz respiratoire qui est guidé à travers le corps de base (1), de sorte qu'une détection in situ des paramètres du gaz respiratoire est rendue possible ; dans lequel
la section de guidage de gaz respiratoire (5) est réalisée sous forme tubulaire et
l'au moins un premier laser (L1), le deuxième laser (L2) et/ou le détecteur (D) sont prévus de manière flexible sur la périphérie de la section de guidage de gaz respiratoire (5), de sorte que la position de l'au moins un premier laser (L1), du deuxième laser (L2) et/ou du détecteur (D) par rapport à la section de guidage de gaz respiratoire (5) peut être modifiée ; et
l'au moins un premier laser (L1), le deuxième laser (L2) et/ou le détecteur (D) sont conçus pour faire varier la plage de mesure du dispositif de mesure (4) en faisant varier la longueur de trajet du premier laser (L1) et/ou du deuxième laser (L2) ;
dans lequel la région intérieure de la section de guidage de gaz respiratoire (5) présente au moins en partie une surface réfléchissante et comprend un réflecteur circulaire et le premier laser (L1) et le deuxième laser (L2) sont conçus pour éclairer la région intérieure de la section de guidage de gaz respiratoire (5).

2. Dispositif selon la revendication 1, dans lequel l'évaluation et/ou l'analyse des paramètres détectés du dispositif de mesure (4) peut être effectuée par le biais de l'unité de calcul (3).

3. Dispositif selon au moins l'une des revendications 1 ou 2, dans lequel le dispositif de mesure (4) comprend un capteur qui présente au moins les deux lasers, dans lequel les paramètres du gaz respiratoire sont déterminés par spectroscopie laser.

4. Dispositif selon la revendication 3, dans lequel le capteur comprend deux lasers qui présentent des longueurs d'onde différentes.

5. Dispositif selon la revendication 3, dans lequel la pression et la température du gaz respiratoire sont déterminées par l'évaluation spectrale du profil linéaire de lignes d'absorption du gaz respiratoire et dans lequel l'évaluation spectrale est effectuée en particulier par l'unité de calcul (3).

6. Dispositif selon au moins l'une des revendications précédentes, dans lequel le dispositif de mesure (4) détecte en tant que paramètre au moins l'un des paramètres suivants : la concentration en CO₂ du gaz respiratoire, la concentration en O₂ du gaz respiratoire, le débit volumique du gaz respiratoire, l'humidité du gaz respiratoire, la température ambiante, la pression du gaz respiratoire.

7. Dispositif selon au moins l'une des revendications précédentes, dans lequel le corps de base (1) peut être fixé à un sujet par le biais de moyens de fixation et l'unité de calcul (3), le dispositif de mesure (4) et l'accumulateur d'énergie (2) sont reçus sur le corps de base (1), dans lequel le corps de base (1) présente la section de guidage de gaz respiratoire pour guider le flux de gaz respiratoire avec une entrée de gaz respiratoire côté sujet et une sortie de gaz respiratoire et dans lequel le capteur (S) est prévu dans la section de guidage de gaz respiratoire.

8. Dispositif selon au moins l'une des revendications précédentes, dans lequel le dispositif obtient l'énergie nécessaire pour le fonctionnement exclusivement par le biais de l'accumulateur d'énergie (2).

9. Dispositif selon au moins l'une des revendications précédentes, dans lequel le dispositif de mesure (4) comprend un capteur infrarouge non dispersif et/ou un capteur à dioxyde de zirconium.

10. Dispositif selon au moins l'une des revendications précédentes, dans lequel un générateur est prévu pour effectuer une récupération d'énergie, de sorte qu'une récupération d'énergie à partir d'impulsions de choc et/ou de chaleur de gaz respiratoire et/ou d'éclairage ambiant est rendue possible.

11. Dispositif selon au moins l'une des revendications précédentes, dans lequel un diaphragme est prévu pour déterminer le débit volumique du gaz respiratoire par le biais d'une pression différentielle dans la section de guidage de gaz respiratoire, lequel diaphragme est prévu entre le premier laser (L1) et le deuxième laser (L2).
